# EUROPEAN PATENT APPLICATION

(11) **EP 2 980 561 A1**
(43) Date of publication of application: **03.02.2016**
(21) Application number: 14773413.1
(22) Date of filing: 19.03.2014
(51) Int. Cl.: G01N 21/3581

(54) **IMAGING SYSTEM**

(30) Priority: 29.03.2013 JP 2013072864; 04.02.2014 JP 2014019269
(71) Applicant: NEC Corporation, Tokyo 108-8001 (JP)
(72) Inventor: ISHI, Tsutomu, Tokyo 108-8001 (JP); SUDOU, Takayuki, Tokyo 108-8001 (JP); TSUBOI, Taku, Tokyo 108-8001 (JP)
(74) Representative: Vossius & Partner Patentanwälte Rechtsanwälte mbB
(86) International application number: PCT/JP2014/058706
(87) International publication number: WO 2014/157431

(57) **Abstract**

The present invention provides an imaging system capable of reducing the generation of interference patterns in a state in which a beam of highly coherent electromagnetic waves is enlarged to a prescribed illumination area and used for illumination by moving the prescribed illumination region within a range of motion that is smaller than the illumination region.

## Description

### Technical Field

The present invention relates to an imaging system and an imaging method using highly coherent electromagnetic waves.

### Background Art

A so-called Terahertz wave in a frequency band of 100 GHz to 10 THz (a wavelength band range from 3 mm to 30 µm) is known as a highly coherent electromagnetic wave. The Terahertz wave is an electromagnetic wave located in a band between infrared light and radio waves, and has property of penetrating through paper, plastic, textile, smoke, water, semiconductors, and the like, and has an absorption spectrum unique to a substance. In addition, the Terahertz wave is safer than X-ray, and is therefore expected to be applied to nondestructive inspection and the like.

Various techniques have been suggested to use Terahertz waves. For example, Patent Literature 1 (PTL 1) discloses a Terahertz wave generation element including an electromagnetic wave component for Terahertz waves, a Terahertz wave detection element, a Terahertz time domain spectroscopy device, and the like. The Terahertz wave generation element disclosed in PTL 1 includes a waveguide including an electro-optic crystal propagating light, an optical coupling member for retrieving a Terahertz wave into a space from a light propagating through the waveguide, and two electrodes. More specifically, cited Document 1 indicates that, by applying an electric field to the waveguide via the electrodes, a change is given to the propagation state of the light propagating through electro-optic crystals, whereby this makes phase matching between the light propagating through the waveguide and the Terahertz wave generated by second-order nonlinear process.

Patent Literature 2 (PTL 2) discloses an imaging device which irradiates electromagnetic waves to an electro-optic crystal from two directions, separates the electromagnetic wave acting each other in the electro-optic crystal into two different polarization components, and capturing an image based on the separated different polarization components. PTL 2 is provided with a signal processing unit for performing difference processing on the signal of the image based on the different polarization components. The imaging device according to PTL 2 can obtain an image having a high S/N ratio.

Further, Patent Literature 3 (PTL 3) includes an electromagnetic wave imaging device including a first optical system for irradiating a pulse-like detection electromagnetic wave to an object to be measured and irradiating a detection electromagnetic wave from the object to an electro-optic crystal, and a second optical system for irradiating a probe wave having a pulse surface inclined with respect to a pulse surface of a detection electromagnetic wave to the electro-optic crystal, wherein a probe wave that has passed through the electro-optic crystal is detected by a camera.

As described above, the Terahertz wave is an electromagnetic wave in a frequency band between infrared light and radio wave, and has a property as light, and therefore, in the following explanation, a term related to light is used for the explanation.

### Citation List

### Patent Literature

PTL 1: Japanese Patent Laid-Open No. 2011-203718
PTL 2: Japanese Patent Laid-Open No. 2012-108117
PTL 3: Japanese Patent Laid-Open No. 2012-122981

### Summary of Invention

### Technical Problem

Any of PTLs 1 to 3 indicates that two electromagnetic waves are irradiated to an electro-optic crystal, and an electromagnetic wave from the electro-optic crystal is captured. However, none of PTLs discloses the problem associated with images obtained by capturing the Terahertz wave.

According to the research of the inventors of the present invention and the like, it is found that, in a case where an object to be measured or a subject is captured by using a highly coherent light source just like the Terahertz wave, it is difficult to recognize the object to be measured or the subject from the captured image. More specifically, in a case where a highly coherent light source is used, interference patterns are captured with illumination light in an overlapping manner with the shape and the darkness of the object or the subject which is originally intended to be captured, and as a result, this makes it difficult to recognize the shape and the darkness of the object or the subject which is originally intended to be captured. For example, it is found that a tiny amount of dust, hair, and the like mixed in the object or the subject is to be identified by an imaging technique using the Terahertz wave, it is difficult to identify a tiny amount of dust, hair, and the like from the image due to the effect of the interference pattern.

It is an object according to an exemplary embodiment of the present invention is to provide an easy and highly effective imaging system that can alleviate generation of an interference pattern.

In addition, the present invention is to provide an interference pattern alleviation method that can alleviate generation of an interference pattern.

### Solution to Problem

According to an aspect of the present invention, an imaging system can be obtained, which includes an optical system enlarging a ray bundle of a highly coherent electromagnetic wave into a predetermined illumination area to illuminate the predetermined illumination area, and movement means for moving the predetermined illumination area within a movement range smaller than the illumination area. In this case, the predetermined illumination area is a flat surface area perpendicular to a ray bundle of a highly coherent electromagnetic wave.

According to another aspect of the present invention, an interference pattern alleviating method can be obtained, which includes illuminating a predetermined illumination area with a highly coherent electromagnetic wave, moving the illumination light within a range smaller than the predetermined illumination area, i.e., causing the illumination light to make swing movement or rotation movement, thus alleviating an interference pattern generated by illuminating the illumination light.

### Advantageous Effects of Invention

In the present invention, generation of an interference pattern generated by illumination light is alleviated, and the shape and the like of an object to be measured or a subject which is originally intended to be captured can be clearly identified.

### Brief Description of Drawings

Fig. 1 is a schematic configuration diagram for explaining an imaging system according to a first exemplary embodiment of the present invention.
Fig. 2 is a schematic configuration diagram for explaining an imaging system according to a second exemplary embodiment of the present invention.
Fig. 3 is a figure for explaining a captured image, in which (A) is a figure in a case where the present invention is not applied, and (B) is a figure in a case where the present invention is applied.
Fig. 4 is a schematic configuration diagram for explaining an imaging system according to a third exemplary embodiment of the present invention.
Fig. 5 is a figure illustrating a polarization, i.e., oscillation direction of an electric field, used as illumination light in Fig. 4.
Fig. 6 is a schematic configuration diagram for explaining an imaging system according to a fourth exemplary embodiment of the present invention.
Fig. 7 is a figure illustrating a polarization, i.e., oscillation direction of an electric field, used as illumination light in Fig. 6.

### Description of Embodiments

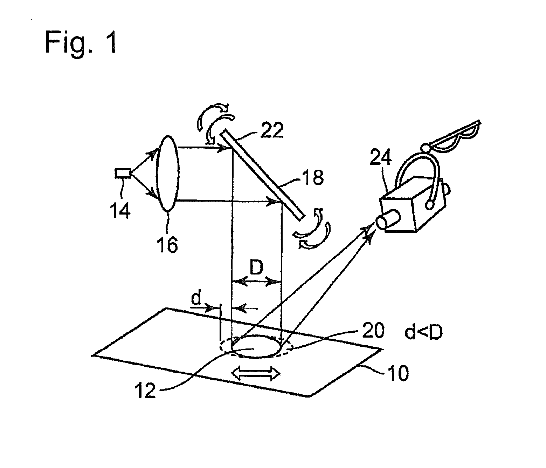

With reference to Fig. 1, an imaging system according to an exemplary embodiment of the present invention is shown. The imaging system shown in Fig. 1 is considered to be used to identify a subject 12, for example, a foreign matter (for example, textile, metal piece, hair) mixed in flour placed on a two-dimensional flat surface 10. In this example, the subject is considered to be placed within a predetermined inspection area on the two-dimensional flat surface 10 determined in advance. In the example shown in the drawing, a light source 14 generating a Terahertz wave as a highly coherent electromagnetic wave and a lens system 16 enlarging the Terahertz wave from the light source 14 are provided, and the lens system 16 emits the Terahertz wave from the light source 14 as a ray bundle enlarged into a two-dimensional-like area.

The ray bundle of the Terahertz wave from the lens system 16 enlarged into the two-dimensional-like area is reflected by a reflection mirror 18, and thereafter, illuminates the inspection area of the two-dimensional flat surface 10 on which the subject 12 is placed. In this case, the two-dimensional flat surface 10 is arranged to be perpendicular to the ray bundle of the Terahertz wave reflected by the reflection mirror 18, and the optical system constituted by the lens system 12 and the reflection mirror 18 is adjusted so that the illumination area 20 of the Terahertz wave illuminates the entire subject area of the two-dimensional flat surface 10. In this case, the optical system is adjusted to that the diameter of the illumination area 20 becomes D.

The imaging system shown in Fig. 1 includes a swing movement mechanism 22 swinging the reflection mirror 18 and an image-capturing device 24 for capturing an image in the illumination area 20. The image-capturing device 24 may be, for example, an infrared light camera, or may be a CCD camera and the like.

The swing movement mechanism 22 for swinging the reflection mirror 18 swings the reflection mirror 18 so that the illumination area 20 of the diameter D does not go beyond the diameter D of the illumination area 20. The swing movement mechanism 22 may be a mechanism for mechanically rotating the reflection mirror 18 in a microscopic range, or may be a mechanism for oscillating the reflection mirror 18 according to an electric signal.

More specifically, the swing movement mechanism 22 may swing the reflection mirror 18 to such a degree that swing movement range d of the illumination area D based on the swing movement of the reflection mirror 18 does not exceed the diameter D of the illumination area 20. More specifically, the swing movement range d is a range smaller than the diameter D of the illumination area 20, and a relationship of d<D or d<<D is satisfied.

In this case, in imaging based on ordinary scanning, a spot-like illumination area scans a range larger than the illumination area. However, as described above, in the imaging based on the ordinary scanning, it takes a long time to perform the image-capturing, and the imaging based on the ordinary scanning is not suitable for discovering a very small foreign matter in a short time. In contrast to the ordinary scanning, the present invention performs imaging by swinging the illumination area 20 in a range smaller than the illumination area 20 while the entire illumination area 20 is illuminated. During the swing movement, the subject is disposed in the illumination area, and the image is captured by the image-capturing device 24 while the subject is not out of the illumination area.

As described above, with the swing movement in the range d smaller than the diameter D of the illumination area 20, interference pattern caused by the imaging is averaged in the swing movement range. As a result, the interference pattern is alleviated in the image captured by the image-capturing device 24, and the interference pattern is less likely to be seen in terms of visual aspect.

In this case, where the diameter D and the swing movement range d of the illumination area 20 are 10 mm and 0.35 mm, respectively, and the interference pattern appearing in the image can be eliminated in terms of visual aspect. This means that when the swing movement range d is set to about 3 to 5% of the diameter D of the illumination area 20, the interference pattern does not explicitly appear in terms of visual aspect.

The imaging system as shown in Fig. 1 uses the image-capturing device 24 to capture the reflection image from the subject arranged in the illumination area 20. However, the present invention is not limited thereto. An image passing through the illumination area 20 may be observed.

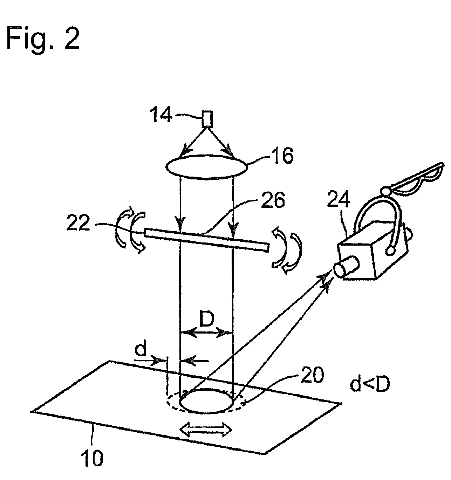

With reference to Fig. 2, an imaging system according to a second exemplary embodiment of the present invention is shown. The imaging system as shown in Fig. 2 is different from Fig. 1 in that, instead of the reflection mirror 18 as shown in Fig. 1, a parallel flat plate 26 that passes a ray bundle of a Terahertz wave from a light source 14 and a lens system 16 is provided. As the parallel flat plate, for example, a glass plate that has transparency for the Terahertz wave can be used as the parallel flat plate 26. In this case, in the ray bundle of the Terahertz wave incident upon the glass plate 26, there is a change in the refraction index of the ray bundle emitted out from the glass plate 26 due to a change in the angle of the ray bundle of the Terahertz wave incident upon the glass plate 26 due to the swing movement. As a result, the illumination area 20 of the diameter D reciprocally moves along the swing movement range d.

The image-capturing device 24 captures the image of the reflection light from the swinging illumination area 20. The illumination area 20 is averaged due to the swing movement of the illumination area 20, and therefore, the interference pattern cannot be observed in the image captured by the image-capturing device 24 in terms of visual aspect, and the image can be obtained so that a foreign matter in the subject can be clearly identified.

The swing movement mechanism 22 shown in Figs. 1 and 2 is attached to the reflection mirror 18 or the glass plate 26. More specifically, the swing movement mechanism 22 may have a mechanism provided at both ends of a central axis of the reflection mirror 18 to rotate and move the reflection mirror 18 or the glass plate 26 within the microscopic range in a galvanometer mirror format in response to an electric signal, for example.

On the other hand, the swing movement mechanism 22 as shown in Fig. 2 may be a mechanism provided in the illumination light path to oscillate the glass plate serving as parallel flat plate 26 about an axis perpendicular to an optical axis. Alternatively, the parallel flat plate 26 may be disposed with an inclination with respect to the optical axis of the illumination light, and the parallel flat plate 26 may be rotated about the optical axis of the illumination light path by using an ultrasonic wave motor or an ordinary motor. As described above, by rotating the parallel flat plate, the illumination area 20 can also be moved in the range d smaller than the illumination area 20. In this case, the range d may be referred to as a movement range with respect to the illumination area. The system as shown in Fig. 2 moves the parallel flat plate 26 in a horizontal state, and therefore, the swing movement mechanism 22 or the driving mechanism for the parallel flat plate 26 can be simplified.

The image-capturing device 24 may also be constituted by, for example, a bolometer-type infrared light image-capturing device in which thermoelectric conversion elements are arranged in a two-dimensional matrix manner.

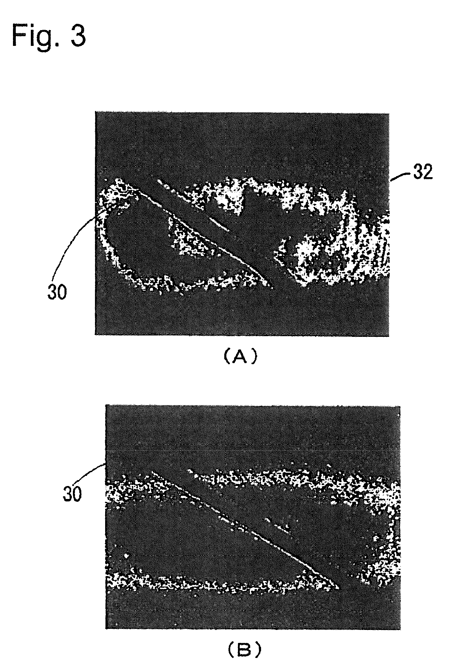

With reference to Figs. 3 (A) and (B), an example of an image captured without applying the present invention is shown in Fig. 3 (A). In this case, Fig. 3 (A) shows a case where the hair 30 is mixed as a foreign matter into flour. In a case where the present invention is not applied as shown in Fig. 3 (A), the interference pattern 32 appears in the background. In such state in which the interference pattern 32 appears, the hair 30 which is the foreign matter is covered with the stripe-shaped interference pattern 32, which makes it difficult to identify the hair 30 in many cases.

On the other hand, Fig. 3 (B) shows an image in a case where the illumination area is swung according to the present invention. As can be clearly understood from Fig. 3 (B), the stripe-shaped interference pattern is eliminated from the background of the hair 30 which is the foreign matter, and only the hair 30 which is the foreign matter is emphasized.

Therefore, it is easy to clearly identify the foreign matter from the image shown in Fig. 3 (B).

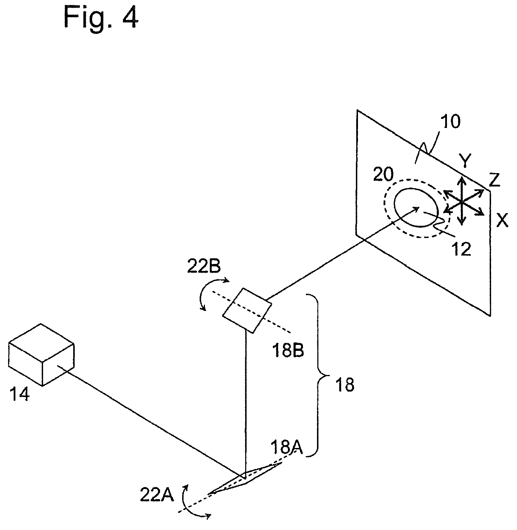

With reference to Fig. 4, an imaging system according to a third exemplary embodiment of the present invention is shown. Although the imaging system as shown in Fig. 4 has the mirror just like the imaging system as shown in Fig. 1, the imaging system as shown in Fig. 4 is different from Fig. 1 in that the imaging system as shown in Fig. 4 includes two mirrors 18A, 18B instead of a single mirror. By using the two mirrors, one of the mirrors, i.e., a mirror 18A, swings about the Z axis in the X direction, and the other of the mirrors, i.e., a mirror 18B, swings about the X axis in the Y direction, so that the illumination area 20 swings in a two-dimensional manner in the two-dimensional flat surface 10. Even with such arrangement, the interference pattern is averaged by the swing movement of the illumination area 20, and therefore, the interference pattern cannot be observed from the image captured by the image-capturing device in terms of visual aspect, and the image can be obtained so that the foreign matter in the subject can be clearly identified.

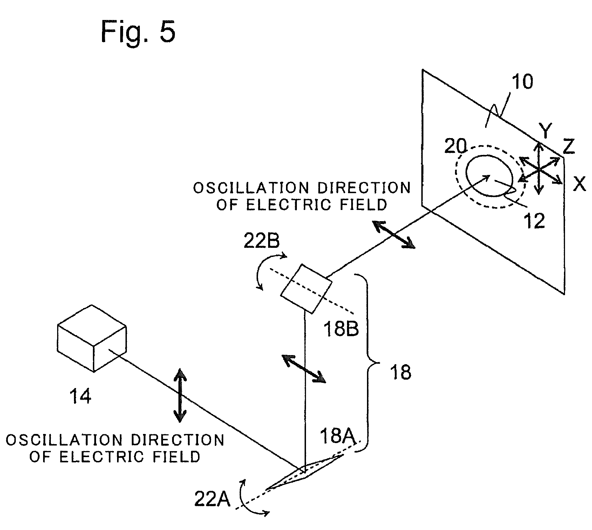

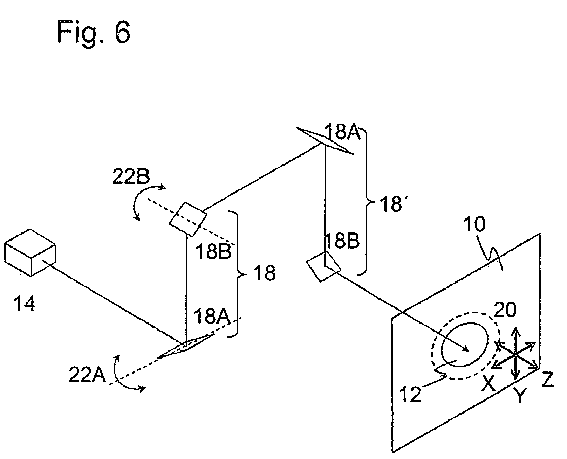

By the way, in a case where a light of polarization, i.e., the electric field oscillates in a particular direction in a regular manner is used as the illumination light in the arrangement as shown in Fig. 4, the polarization direction may change in a direction different from the intended direction. This occurs, for example, in a case where the incidence planes of the two mirrors (a surface perpendicular to the reflection plane and including the incident light beam and the reflection light beam) are perpendicular to each other. This will be explained with reference to Fig. 5, it is understood that the oscillation direction of the electric field oscillates in the vertical direction with respect to the travelling direction, but after passing the two mirrors, the oscillation direction of the electric field oscillates in the horizontal direction with respect to the travelling direction. An imaging system according to a fourth exemplary embodiment of the present invention is shown in Fig. 6. The imaging system as shown in Fig. 6 includes two pairs of mirror pairs 18 and 18' of which incidence planes are perpendicular to each other. Among them, the mirror pair 18 (18A and 18B) include the swing movement means 22A, 22B, and the other of the mirror pairs, i.e., the mirror pair 18', does not include the swing movement means.

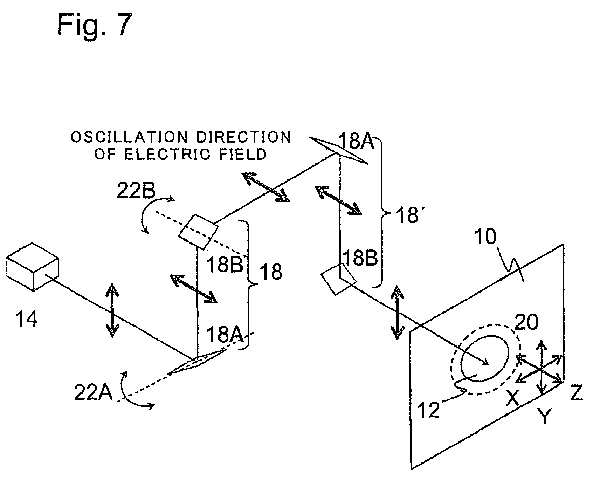

Fig. 7 illustrates oscillation directions of the electric field in Fig. 6. The light that was, at first, oscillating in the vertical direction with respect to the travelling direction oscillates in the horizontal direction with respect to the travelling direction after the light passes the mirror pair 18, and again, after the light passes another mirror pair 18', the light oscillates in the vertical direction with respect to the travelling direction. As described above, in order to average the interference pattern by the swing movement of the illumination area 20, it is obvious that it is sufficient to swing at least one of the mirror pairs.

According to an imaging system of the present invention explained above, the subject can be illuminated with a ray bundle of illumination light which is larger than diffraction limitation by orders of magnitude without applying complicated measures for preventing reflection and measures for preventing diffraction to the illumination optical system and the subject. Therefore, with the present invention, there is an advantage in that a range over a wide field of vision can be captured at a time, and the image-capturing quality can be greatly improved, and the image-capturing process can be performed at a high speed.

### Industrial Applicability

In the above exemplary embodiment, only the case where the Terahertz wave is used has been explained, but the present invention can also be applied to an imaging system using a highly coherent visible light laser light and the like. In this case, the image-capturing device does not need to use an infrared light image-capturing device, and the image-capturing device may be a CCD camera.

Hereinafter, the features of the present invention will be described in Supplemental notes.

### [Supplemental note 1]

An imaging system comprising an optical system enlarging a ray bundle of a highly coherent electromagnetic wave into a predetermined illumination area to illuminate the predetermined illumination area, and movement means for moving the predetermined illumination area within a movement range smaller than the illumination area.

### [Supplemental note 2]

An imaging system, wherein the illumination area is a flat surface perpendicular to the ray bundle of the illumination light incident upon the illumination area.

### [Supplemental note 3]

The imaging system comprising image-capturing means for capturing an image of a subject arranged in the illumination area.

### [Supplemental note 4]

The imaging system, wherein the highly coherent electromagnetic wave is an electromagnetic wave of a Terahertz band.

### [Supplemental note 5]

The imaging system, wherein the movement means is swing movement means for swinging the illumination area within the movement range capable of alleviating an interference pattern generated by the illumination.

### [Supplemental note 6]

The imaging system, wherein the optical system includes a light source emitting the highly coherent electromagnetic wave, a lens system for enlarging the highly coherent electromagnetic wave to the predetermined illumination area, and an optics system for guiding the ray bundle from the lens system to the illumination area.

### [Supplemental note 7]

The imaging system, wherein the optics system for guiding to the illumination area includes a mirror for reflecting the ray bundle from the lens system to the illumination area.

### [Supplemental note 8]

The imaging system, wherein the swing movement means is a mechanism in a galvanometer mirror format for swinging the mirror within the movement range smaller than the illumination area.

### [Supplemental note 9]

The imaging system, wherein the optics system for guiding to the illumination area includes a parallel flat plate inserted into an illumination light path extending from the lens system.

### [Supplemental note 10]

The imaging system, wherein the movement means is swing movement means for swinging the parallel flat plate about an axis perpendicular to an optical axis within the movement range.

### [Supplemental note 11]

The imaging system, wherein the swing movement means is rotation means for rotating the parallel flat plate about an axis perpendicular to the optical axis.

### [Supplemental note 12]

An interference pattern alleviating method comprising: illuminating a predetermined illumination area with a highly coherent electromagnetic wave, moving the illumination light within a range smaller than the predetermined illumination area, and thus alleviating an interference pattern generated by illuminating the illumination light.

### [Supplemental note 13]

An imaging system comprising an optical system for forming an illumination area of a ray bundle of a highly coherent electromagnetic wave on a two-dimensional flat surface, and movement means for relatively moving at least one of the two-dimensional flat surface and the optical system within a movement range smaller than the illumination area.

### Reference signs List

- 10: two-dimensional flat surface 10
- 12: subject
- 14: light source
- 16: lens system
- 18: reflection mirror
- 18A: mirror
- 18B: mirror
- 20: illumination area
- 22: swing movement mechanism
- 22A: swing movement mechanism
- 22B: swing movement mechanism
- 24: image-capturing device
- 26: parallel flat plate

This application is based upon and claims the benefit of priority from Japanese patent application No. 2013-072864 filed on March 29, 2013 and Japanese patent application No. 2014-019269 filed on February 4, 2014, the disclosure of which is incorporated herein in its entirety by reference.

## Claims

1. An imaging system comprising: an optical system enlarging a ray bundle of a highly coherent electromagnetic wave into a predetermined illumination area to illuminate the predetermined illumination area, and movement means for moving the predetermined illumination area within a movement range smaller than the illumination area.

2. An imaging system, wherein the illumination area is a flat surface perpendicular to the ray bundle of the illumination light incident upon the illumination area.

3. The imaging system according to claim 1 or 2, comprising: image-capturing means for capturing an image of a subject arranged in the illumination area.

4. The imaging system according to any one of claims 1 to 3, wherein the highly coherent electromagnetic wave is an electromagnetic wave of a Terahertz band.

5. The imaging system according to any one of claims 1 to 4, wherein the movement means is swing movement means for swinging the illumination area within the movement range capable of alleviating an interference pattern generated by the illumination.

6. The imaging system according to any one of claims 1 to 5, wherein the optical system includes a light source emitting the highly coherent electromagnetic wave, a lens system for enlarging the highly coherent electromagnetic wave to the predetermined illumination area, and an optics system for guiding the ray bundle from the lens system to the illumination area.

7. The imaging system according to claim 5, wherein the optics system for guiding to the illumination area includes a mirror for reflecting the ray bundle from the lens system to the illumination area.

8. The imaging system according to claim 7, wherein the mirror includes a pair of mirrors of which incidence planes are perpendicular to each other.

9. The imaging system according to claim 8, wherein each of the mirrors includes the swing movement means.

10. The imaging system according to claim 7, comprising: a plurality of pairs of mirrors of which incidence planes are perpendicular to each other, and wherein
at least a pair of mirrors includes the swing movement means, and the other thereof does not include the swing movement means.

11. The imaging system according to any one of claims 5 to 7, wherein the swing movement means is a mechanism in a galvanometer mirror format for swinging the mirror within the movement range smaller than the illumination area.

12. The imaging system according to claim 6, wherein the optics system for guiding to the illumination area includes a parallel flat plate inserted into an illumination light path extending from the lens system.

13. The imaging system according to claim 12, wherein the movement means is swing movement means for swinging the parallel flat plate about an axis perpendicular to the optical axis within the movement range.

14. The imaging system according to claim 12, wherein the swing movement means is rotation means for rotating the parallel flat plate about an axis perpendicular to the optical axis.

15. An interference pattern alleviating method comprising: illuminating a predetermined illumination area with a highly coherent electromagnetic wave, moving the illumination light within a range smaller than the predetermined illumination area, and thus alleviating an interference pattern generated by illuminating the illumination light.

16. An imaging method comprising: illuminating a predetermined illumination area with a highly coherent electromagnetic wave, moving the illumination light within a range smaller than the predetermined illumination area, and detecting a foreign matter in a subject located in the illumination area by illuminating the illumination light.
